# EUROPEAN PATENT APPLICATION

(11) **EP 1 738 779 A1**
(43) Date of publication of application: **03.01.2007**
(21) Application number: 06445056.2
(22) Date of filing: 28.06.2006
(51) Int. Cl.: A61L 15/18, A61F 13/38

(54) **Swab device and kit for the delivery of blood clotting materials to a wound site**

(30) Priority: 30.06.2005 US 174255
(71) Applicant: Horn, Jeffrey L., Rocky Hill, CT 06067 (US); Huey, Raymond J., Orange, CT 06477 (US)
(72) Inventor: Horn, Jeffrey L., Rocky Hill, CT 06067 (US); Huey, Raymond J., Orange, CT 06477 (US)
(74) Representative: Israelsson, Stefan

(57) **Abstract**

A swab insertable directly into a wound or into a body cavity to treat a wound comprises an elongated member having material attached at one or both ends thereof. The material may be or may include a molecular sieve material such as zeolite. A swab for promoting the clotting of blood at a wound site comprises a frangible straw containing a molecular sieve material in particulate form and at least one contact pad attached to the straw. A kit for the treatment of a bleeding wound includes a swab (which may be a conventional swab or a swab having molecular sieve material attached at one or both ends), a carrier material, and a molecular sieve material in particulate form.

## Description

### Technical Field

The present invention relates generally to blood clotting devices and, more particularly, to swabs and swab kits that facilitate the application of blood clotting materials to wound sites. The present invention is also directed to methods for the use of such devices for controlling bleeding.

### Background of the Invention

Blood is a liquid tissue that includes red cells, white cells, corpuscles, and platelets dispersed in a liquid phase. The liquid phase is referred to as plasma and includes acids, lipids, solublized electrolytes, and proteins. The proteins are suspended in the liquid phase and can be separated out of the liquid phase by any of a variety of methods such as filtration, centrifugation, electrophoresis, and immunochemical techniques. One particular protein suspended in the liquid phase is fibrinogen. When bleeding occurs, the fibrinogen reacts with water and thrombin (an enzyme) to form fibrin, which is insoluble in blood and polymerizes to form clots.

In a wide variety of circumstances humans (as well as animals) can be wounded, thereby resulting in bleeding. In some circumstances, the wound and the bleeding are minor, and normal blood clotting functions in addition to the application of simple first aid are all that is required. Unfortunately, however, in other circumstances substantial bleeding can occur. These situations usually require specialized equipment and materials as well as personnel trained to administer appropriate aid. If such aid is not readily available, excessive blood loss can occur. When bleeding is severe, sometimes the immediate availability of equipment and trained personnel is still insufficient to stop the flow of blood in a timely manner.

In an effort to address the above-described problems, materials and devices have been developed for controlling excessive bleeding in situations where conventional aid is unavailable or less than optimally effective. Although these materials and devices have been shown to be somewhat successful, they are sometimes not effective enough for traumatic wounds and tend to be expensive. Furthermore, these materials can be difficult to apply as well as remove from a wound.

In situations in which wounds that extend deep beneath the skin are inflicted (for example, punctures or lacerations resulting from stabbings), arteries and/or internal organs may be adversely affected. First aid may be administered with some success to treat such a wound at the surface, but treatment below the surface of the skin may be more difficult. Furthermore, when a sub-dermal wound is inflicted, the surrounding muscle tends to close over the wound. Particularly with regard to punctures or deep lacerations, substantial amounts of blood may be unnecessarily lost before the wound can be properly treated and closed by trained personnel.

In situations in which blood emanates from an orifice in a person's body (e.g., during a nosebleed) or within a body cavity (e.g., in the mouth during dental procedures), treatment of the wound may be difficult due to the inability to easily access the wound site. Treatment in such cases, even if effective, often results in the use of invasive techniques that cause discomfort to the patient.

Based on the foregoing, it is the general object of the present invention to provide devices for controlling bleeding and methods of their use that overcome or improve upon the prior art.

### Summary of the Invention

According to one aspect, the present invention resides in a swab that may be inserted directly into a wound or into a body cavity to treat a wound. As used herein, the word "swab" should be broadly construed to encompass elongated members having wads of material attached at one or both ends thereof. The elongated members may be sticks, straws, wires, flexible rods, or the like, and the wads attached at the end(s) thereof comprise material that is used to apply compounds such as blood clotting agents to wounds and/or material to facilitate the removal of debris from a wound. The material preferably comprises a molecular sieve material (e.g., a zeolite) used as a hemostatic agent to facilitate the clotting of blood. Preferably, the molecular sieve material is retained in a mesh structure of the material.

In another aspect of the present invention, a swab for promoting the clotting of blood at a wound site includes a frangible straw containing a molecular sieve material in particulate form and at least one contact pad attached to the straw. To treat a bleeding wound with such a swab, the frangible straw is broken open, the molecular sieve material is poured onto the wound, and the contact pad is used to facilitate contact between the molecular sieve material and the points at which blood emanates.

In yet another aspect, the present invention resides in a kit for the treatment of a bleeding wound. Such a kit includes a swab (which may be a conventional swab or any of the swabs disclosed herein), a carrier material, and a molecular sieve material in particulate form. The carrier material is applied to the swab preferably by dipping or swiping the contact pad of the swab into the carrier material, and molecular sieve material is applied to the carrier material by dipping the swab/carrier material into the molecular sieve material. The swab is then inserted into the wound such that the molecular sieve material comes into contact with blood, thereby facilitating the clotting of the blood.

Surprisingly, the depth of bleeding puncture wounds or lacerations is often misjudged by emergency services personnel when treating a victim, particularly when the victim has other injuries that appear to be more severe. An advantage of the present invention is that puncture wounds or lacerations can be treated at the point(s) at which blood emanates and not solely at the skin surface adjacent to the wound site. By employing the disclosed devices such that zeolite (or other molecular sieve) material in the devices can reach wounds that are difficult to access, bleeding can be stopped throughout the wound and not merely at the visible portions of the tissue.

Another advantage of the present invention is that the proper dose of molecular sieve material can be readily applied to a bleeding wound. Particularly when the device is a swab or the like in which zeolite material is incorporated, the device can be readily removed from sterilized packaging and used to treat the wounds directly at the points from which blood emanates to facilitate clotting of the blood without spilling powder or pellets outside the wound area. Guesswork, estimation, or calculation of the amounts of molecular sieve material for application to a bleeding wound is eliminated. Accordingly, little or no molecular sieve material is wasted.

### Brief Description of the Drawings

FIG. 1 is a perspective view of a swab incorporating a molecular sieve material, of the present invention.
FIG. 2 is a perspective view of a swab of the present invention having two contact pads.
FIG. 3 is a perspective view of a contact pad of the swab of the present invention.
FIG. 4 is a schematic representation of a permeable layer of the contact pad of the swab.
FIG. 5 is a side view of one embodiment of the permeable layer.
FIG. 6 is a side view of another embodiment of the permeable layer.
FIG. 7 is a perspective view of another embodiment of a swab having a molecular sieve material incorporated into a contact pad thereof.
FIG. 8 is a perspective view of a swab having molecular sieve material incorporated into two contact pads of the swab.
FIG. 9 is a cross sectional view of the contact pad of the swab of FIG. 7.
FIG. 10 is a perspective view of another embodiment of a swab of the present invention.
FIG. 11 is a side view of one end of the swab of FIG. 10.

### Detailed Description of the Preferred Embodiments

Disclosed herein are devices, kits, and methods for delivering materials to wounds to promote the clotting of blood and the dressing of the wounds. The devices can be inserted into wounds to maintain contact between hemostatic (blood clotting) agents and bleeding wound sites of the victim such that the agents incorporated into the devices are contact the tissue of the wound to minimize or stop blood flow by absorbing at least portions of the liquid phases of the blood, thereby promoting clotting. The devices encompass swabs that incorporate the blood clotting agents in the form of molecular sieve materials. In any device, the blood clotting agent is preferably a particulate molecular sieve material that can be maintained in direct contact with blood emanating from a wound through a permeable layer.

The molecular sieve material used in the present invention may be a synthetic polymer gel, cellulose material, porous silica gel, porous glass, alumina, hydroxyapatite, calcium silicate, zirconia, zeolite, or the like. Exemplary synthetic polymers include, but are not limited to, stylene-divinylbenzene copolymer, cross-linked polyvinyl alcohol, cross-linked polyacrylate, cross-linked vinyl ether-maleic anhydride copolymer, cross-linked stylene-maleic anhydride copolymer or cross-linked polyamide, and combinations thereof.

The molecular sieve material is preferably a zeolite. Other molecular sieve materials that may be used include, but are not limited to, faujasite. As used herein, the term "zeolite" refers to a crystalline form of aluminosilicate having the ability to be dehydrated without experiencing significant changes in the crystalline structure. The zeolite may include one or more ionic species such as, for example, calcium and sodium moieties. Typically, the zeolite is a friable material that is about 90% by weight calcium and about 10% by weight sodium. The calcium portion contains crystals that are about 5 angstroms in size, and the sodium portion contains crystals that are about 4 angstroms in size. The preferred molecular structure of the zeolite is an "A-type" crystal, namely, one having a cubic crystalline structure that defines round or substantially round openings.

The zeolite may be mixed with or otherwise used in conjunction with other materials having the ability to be dehydrated without significant changes in crystalline structure. Such materials include, but are not limited to, magnesium sulfate, sodium metaphosphate, calcium chloride, dextrin, a polysaccharide, combinations of the foregoing materials, and hydrates of the foregoing materials.

Zeolites for use in the disclosed applications may be naturally occurring or synthetically produced. Numerous varieties of naturally occurring zeolites are found as deposits in sedimentary environments as well as in other places. Naturally occurring zeolites that may be applicable to the compositions described herein include, but are not limited to, analcite, chabazite, heulandite, natrolite, stilbite, and thomosonite. Synthetically produced zeolites that may also find use in the compositions and methods described herein are generally produced by processes in which rare earth oxides are substituted by silicates, alumina, or alumina in combination with alkali or alkaline earth metal oxides.

Various materials may be mixed with, associated with, or incorporated into the zeolites to maintain an antiseptic environment at the wound site or to provide functions that are supplemental to the clotting functions of the zeolites. Exemplary materials that can be used include, but are not limited to, pharmaceutically-active compositions such as antibiotics, antifungal agents, antimicrobial agents, anti-inflammatory agents, analgesics (e.g., cimetidine, chloropheniramine maleate, diphenhydramine hydrochloride, and promethazine hydrochloride), bacteriostatics, compounds containing silver ions, and the like. Other materials that can be incorporated to provide additional hemostatic functions include ascorbic acid, tranexamic acid, rutin, and thrombin. Botanical agents having desirable effects on the wound site may also be added.

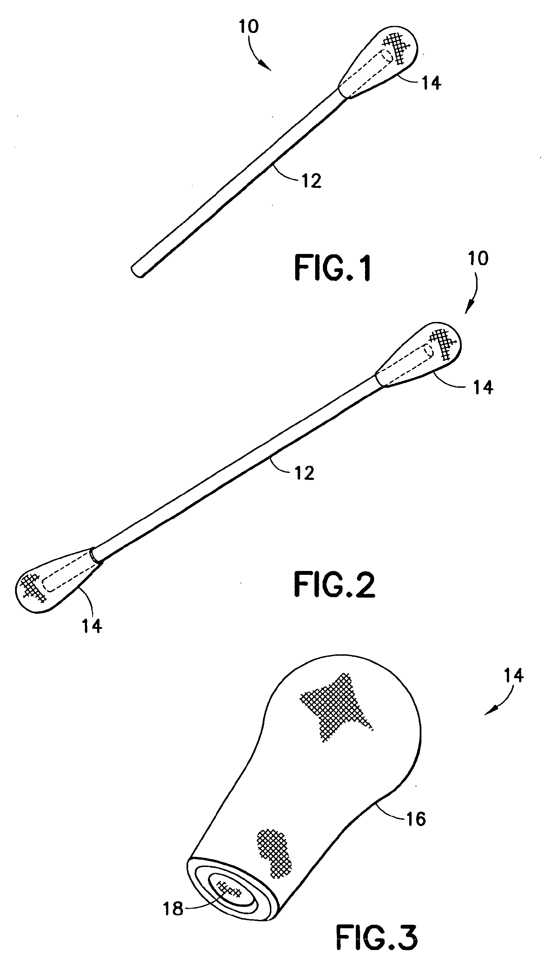

In one embodiment of the present invention shown in FIGS. 1 and 2, a swab that can be inserted into a wound or into a body cavity is shown at reference numeral 10. The swab 10 comprises a stick or an elongated support 12 having a contact pad 14 attached at one end (FIG. 1) or both ends (FIG. 2). During use, a blood clotting agent in the form of a molecular sieve material (hereinafter "zeolite") is incorporated into the contact pad 14 and is maintained in contact with the wound site to facilitate the clotting of blood directly at the wound site. Although the swab 10 preferably comprises the elongated support 12, any elongated member may be substituted therefor. Exemplary elongated members include, but are not limited to, sticks, straws, wires, flexible rods, and the like. Sealed packaging (not shown) provides a sterile environment for storing the swab 10 until it can be used.

Referring now to FIG. 3, in one embodiment the contact pad 14 is defined by a layer of permeable material 16 that is rolled upon itself or otherwise wadded so as to define an open end 18. Preferably, the rolling or wadding of the permeable material 16 is such that the contact pad 14 defines a bulbous portion opposite the open end 18. The contact pad 14 is attached to the elongated support at the open end 18 using any suitable means such as, for example, adhesives, frictional fit methods, heat welding techniques, combinations of the foregoing, and the like.

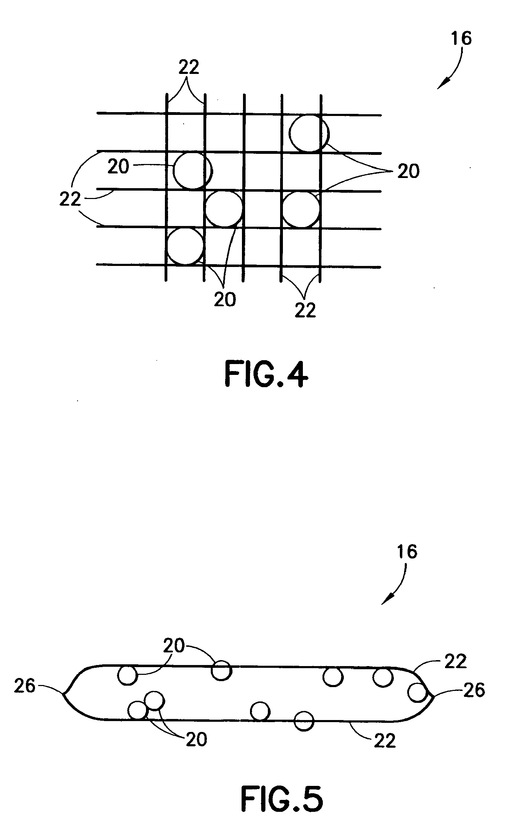

Referring now to FIG. 4, the permeable material 16 is preferably a mesh structure having zeolite particles retained therein. The mesh structure defines openings that are capable of allowing blood to flow therethrough. As illustrated, only a few zeolite particles 20 are shown retained in the mesh structure.

The permeable material 16 is defined by interconnected strands, filaments, or strips of material. The strands 22 can be interconnected in any one or a combination of manners including, but not limited to, being woven into a gauze, intertwined, integrally-formed, and the like. Preferably, the interconnection is such that the permeable material 16 can flex while substantially maintaining the dimensions of the openings defined by the interconnection of the strands 22. The material from which the strands 22 are fabricated may be a polymer (e.g., nylon, polyethylene, polypropylene, polyester, or the like), metal, fiberglass, or an organic substance (e.g., cotton, wool, silk, or the like).

The openings defined by the permeable material 16 are dimensioned to retain the zeolite particles 20 but to accommodate the flow of blood therethrough. Because the zeolite particles 20 may be tightly packed into the permeable material 16, the particles may partially extend through the openings. However, it is not a requirement of the present invention that the zeolite particles 20 protrude through the openings of the permeable material 16. If the zeolite particles 20 do extend through the openings, the particles are able to directly contact tissue to which the permeable material 16 is applied. Thus, blood emanating from the tissue immediately contacts the zeolite particles 20, and the water phase thereof is wicked into the zeolite material, thereby facilitating the clotting of the blood.

The zeolite particles 20 are substantially spherical or irregular in shape (e.g., balls, beads, pellets, granules, rods, flakes, chips, or the like, as well as combinations thereof) and about 0.2 millimeters (mm) to about 10 mm in diameter, preferably about 1 mm to about 7 mm in diameter, and more preferably about 2 mm to about 5 mm in diameter. In any embodiment (balls, beads, pellets, etc.), less particle surface area is available to be contacted by blood as the particle size is increased. Therefore, the rate of clotting can be controlled by varying the particle size. Furthermore, the adsorption of moisture (which also has an effect on the exothermic effects of the zeolite) can also be controlled.

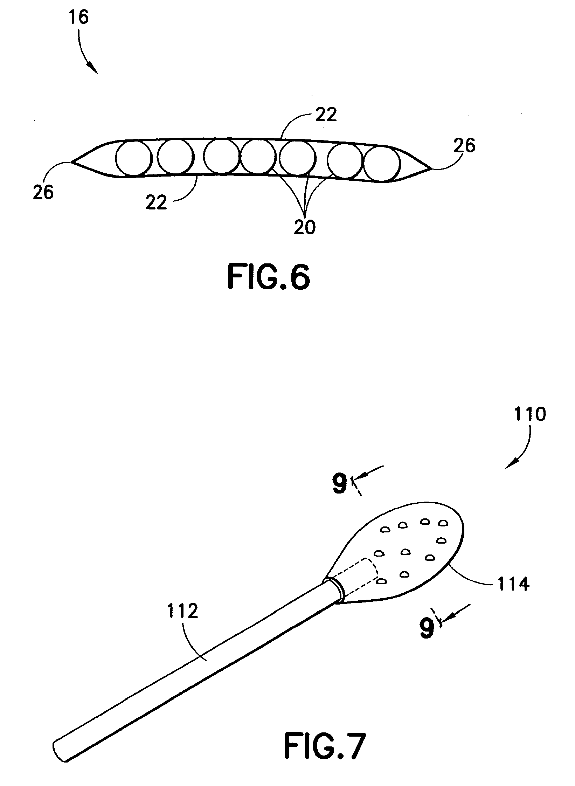

Referring to FIGS. 5 and 6, the permeable material 16 is preferably defined by two layers of interconnected strands 22 between which the zeolite particles 20 are captured. Upper and lower layers of strands 22 are connected along the edges thereof to define seams 26, which may be formed by the mechanical attachment (tying) of the strands 22 of the upper mesh and the lower mesh. The strands 22 of the upper and lower meshes may also be adhesively attached, stitched together, welded, or the like. As shown in FIG. 5 (in which only a few zeolite particles are shown), the spacing between the strands 22 of the upper mesh and lower mesh may be such that there are several layers of ordered zeolite particles 20 or such that the particles are randomly packed. In FIG. 6, the upper mesh and lower mesh may be arranged such that a single layer of zeolite particles 20 is captured. In any arrangement of zeolite particles 20 (ordered layers, random packing, or single layer), the mesh defined by the permeable material 16 can be wrapped or otherwise wadded to form the contact pad, which can be attached to the elongated support to form the swab.

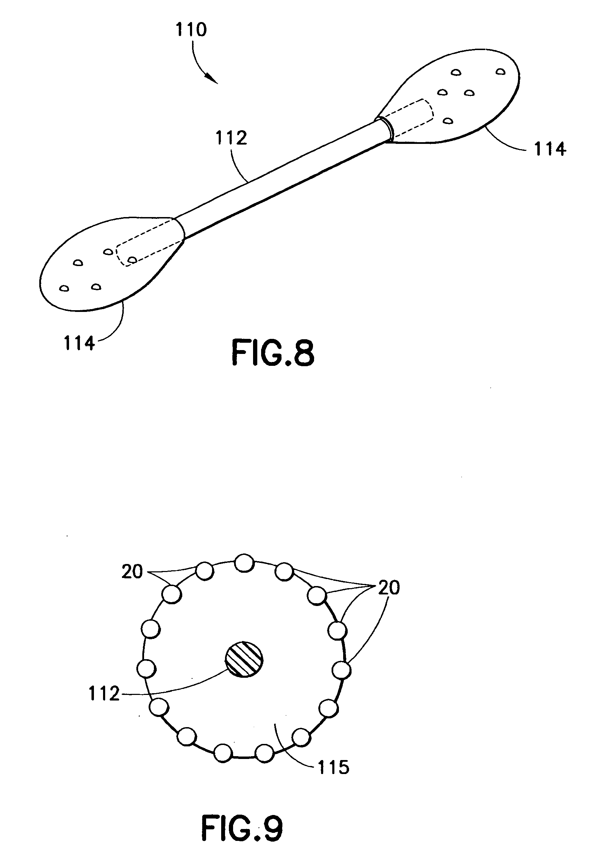

In another embodiment of the present invention as shown with reference to FIGS. 7-9, a swab 110 comprises an elongated support 112 having a contact pad 114 into which a blood clotting agent is incorporated is attached at one end (FIG. 7) or both ends (FIG. 8) of the elongated support. The contact pad 114 may be a substrate 115 having zeolite particles 20 (or some other molecular sieve material) embedded therein, adhesively mounted thereto, or otherwise secured to the substrate such that the substrate and the particles form a wound-engaging surface. The material of the substrate 115 may be any type of material that is bio-compatible with the tissue of a wound and that is capable of retaining zeolite material therein, particularly in the moist environment of a bleeding wound. As above, the zeolite particles 20 may be pellets, granules, beads, rods, flakes, chips, balls, or the like, as well as combinations of the foregoing. As shown in FIG. 9, the zeolite particles 20 are embedded in the substrate 115 to such a depth so as to allow the zeolite particles to protrude above the uppermost surface of the substrate to contact the tissue of the wound.

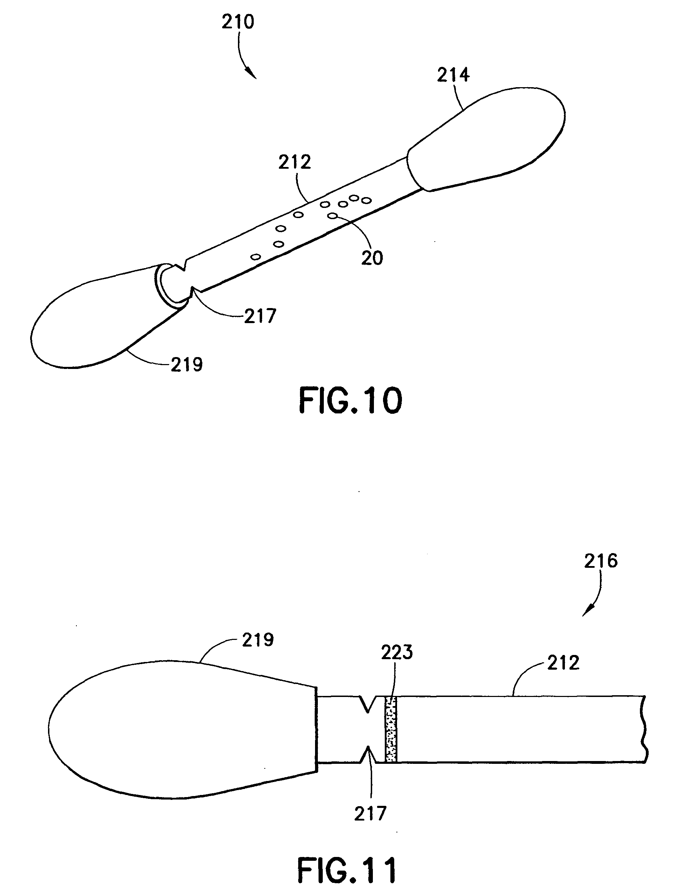

In yet another embodiment of the present invention as shown with reference to FIG. 10, a swab 210 comprises a frangible straw 212 having a contact pad 214 attached at one end. The straw 212 is fabricated from any material having sufficient strength to allow the straw to be cleanly broken without splinters, shards, or small particles separating from the two pieces. Inside the straw 212 are zeolite particles 20 (or any other suitable molecular sieve material) that, upon the breaking of the straw, can be applied to a bleeding wound. Preferably, the straw 212 is sealed at both ends thereof. The contact pad 214 may be any absorbent material that can be wadded, cut, or otherwise formed and attached at the end of the straw 212 such as, for example, muslin, linen, or open-cell foam material. The contact pad 214 may also, as above, comprise a permeable material defined by interconnected strands (e.g., a gauze) that may or may not retain zeolite particles.

In any embodiment of swab 210, the straw 212 is fabricated to have a point of stress that is calculated to fail upon the application of a predetermined amount of force. This point of stress, which is shown at 217 and is hereinafter referred to as "stress point 217," may be formed as a channel, a groove, one or more notches, or the like in a surface of the straw 212. Preferably, the stress point 217 is positioned proximate the end of the straw 212 opposite the end having the contact pad 214. Referring to both FIGS. 10 and 11, a second contact pad 219 is attached at the end of the straw 212 at which the stress point 217 is located to allow leverage to be applied to break the straw at the stress point. An indicator mark 223 is located on or adjacent the stress point 217 to indicate where the straw 212 should be broken to release the zeolite particles 20 contained therein.

To use the swab 210, a user would grasp the swab to support the straw 212 at the ends thereof and apply pressure at the stress point 217. In short, the user attempts to bend or twist the straw 212 at the stress point 217. Upon attempting to bend or twist the straw 212 and breaking the straw into two portions, the zeolite particles 20 contained within the straw can be poured onto a bleeding wound. The shorter end of the swab 210 (i.e., the end with the second contact pad 219) should be discarded. The longer end of the swab 210 (i.e., the end with the contact pad 214) can be used to work the zeolite particles 20 into the wound to facilitate contact between the zeolite particles and the tissue at the points from
which blood emanates.

In yet another embodiment of the present invention, a kit for the application of a molecular sieve material to a bleeding wound may comprises a swab, which may be a conventional swab or any of the swabs as described above, a carrier material, and zeolite particles. The carrier material may be any semi-solid ointment-type composition comprising petrolatum, fatty material such as lanolin, a combination thereof, or the like. Preferably, the carrier material is U.S.P. grade and bio-compatible with human tissue. Each of the swab, the carrier material, and the zeolite particles are packaged separately. One type of packaging for the carrier material and the zeolite particles may be foil packets.

To use the swab kit, a user would grasp the swab, immerse the contact pad end of the swab into the carrier material or at least swipe the swab into the carrier material, dip the swab having the carrier material thereon into the zeolite particles, and insert the swab into the wound to facilitate contact between the zeolite particles and the tissue of the wound at the points from which blood emanates. In addition to being used to treat puncture wounds or lacerations, such an application is especially useful in situations
where a user suffers from a nosebleed, ear bleeding, gingival or other mouth bleeding, or a bleeding hemorrhoid.

In the preparation of zeolite material (i.e., formation of the material into particle form) for the devices of the present invention, an initial level of hydration of the zeolite may be controlled by the application of heat to the zeolite material either before or after the material is formed into particles. However, it has also surprisingly been found that as the particle size of the zeolite is increased, the moisture content has less of a correlative effect on any exothermia produced as the result of mixing the particlized zeolite in blood. As such, formation of the zeolite material into the zeolite particles may be by extrusion, milling, casting, or the like.

Although this invention has been shown and described with respect to the detailed embodiments thereof, it will be understood by those of skill in the art that various changes may be made and equivalents may be substituted for elements thereof without departing from the scope of the invention. In addition, modifications may be made to adapt a particular situation or material to the teachings of the invention without departing from the essential scope thereof. Therefore, it is intended that the invention not be limited to the particular embodiments disclosed in the above detailed description, but that the invention will include all embodiments falling within the scope of the above description.

## Claims

1. A swab (10, 110, 210) for promoting the clotting of blood, including:
an elongated support (12, 112); and **characterized by**
at least one contact pad (14, 114, 214) having a molecular sieve material in particulate form retained therein attached to an end of said elongated support (12, 112);
wherein when treating a bleeding wound, insertion of said swab (10, 110, 210) into said bleeding wound causes at least a portion of said molecular sieve material to come into contact with blood.

2. The swab (10, 110,210) of claim 1, wherein said molecular sieve material is a zeolite.

3. The swab (10, 110, 210) of claim 2, wherein said zeolite includes particles (20) having diameters of about 0.2 mm to about 10 mm.

4. The swab (10, 110, 210) of claim 1, wherein said contact pad (14, 114, 214) is a permeable material defined by a mesh structure having openings therein sized to retain said molecular sieve material, said openings being sized such that blood is able to come into contact with said molecular sieve material through said openings.

5. The swab (10, 110, 210) of claim 4, wherein at least one of said particles (20) of said particulate molecular sieve material protrudes through one of said openings.

6. The swab (10, 110, 210) of claim 4, wherein said mesh structure is defined by two or more layers of interconnected strands (22), each layer being connected to an adjacent layer at the edges thereof; and
wherein said molecular sieve material is captured between said adjacent layers.

7. The swab (10, 110,210) of claim 1, wherein said contact pad (14, 114, 214) includes a substrate (115) having said molecular sieve material secured thereto.

8. The swab (10, 110, 210) of claim 7, wherein said molecular sieve material is embedded into said substrate (115).

9. The swab (10, 110, 210) of claim 7, wherein said molecular sieve material is adhesively mounted to said substrate (115).

10. A swab (10, 110, 210) for promoting the clotting of blood, including:
a frangible straw (212); **characterized by**
a molecular sieve material in particulate form contained within said straw (212); and
at least one contact pad (14, 114, 214) attached to said straw (212);
wherein said straw (212) is breakable to release said molecular sieve material for application to a bleeding wound.

11. The swab (10, 110, 210) of claim 10, wherein said molecular sieve material is a zeolite.

12. The swab (10, 110, 210) of claim 11, wherein said zeolite includes particles (20) having diameters of about 0.2 mm to about 10 mm.

13. The swab (10, 110, 210) of claim 10, wherein said straw (212) is breakable at a stress point (217) located on said straw (212).

14. The swab (10, 110,210) of claim 13, wherein said stress point (217) is formed by at least one of a notch, groove, or channel on said straw (212).

15. The swab (10, 110, 210) of claim 13, further including an indicating mark (223) at said stress point (217) to aid in the identification of said stress point (217) for breaking said straw (212).

16. A kit for the treatment of a bleeding wound, said kit including:
a swab (10, 110, 210);
a carrier material; and **characterized by**
a molecular sieve material in particulate form;
wherein said swab (10, 110, 210) is contactable with said carrier material, said carrier material is contactable with said molecular sieve material, and said molecular sieve material is applicable to said bleeding wound.

17. The kit of claim 16, wherein said carrier material is a composition having a component selected from the group consisting of petrolatum, lanolin, and combinations of the foregoing.

18. The kit of claim 16, wherein said molecular sieve material is a zeolite.

19. The kit of claim 18, wherein said zeolite includes particles (20) having diameters of about 0.2 mm to about 10 mm.

20. The kit of claim 16, wherein each of said swab (10, 110, 210), said carrier material, and said molecular sieve material are packaged separately.

21. The kit of claim 16, wherein said carrier material is packaged in a foil packet.

22. The kit of claim 16, wherein said molecular sieve material is packaged in a foil packet.

23. A method of treating a bleeding wound, **characterized by** the steps of:
providing a frangible straw (212) having a molecular sieve material contained therein and a contact pad (14, 114, 214) attached thereto;
breaking said straw (212);
pouring said molecular sieve material onto said bleeding wound; and
inserting said contact pad (14, 114, 214) into said bleeding wound to facilitate contact between said molecular sieve material and blood.

24. A method of treating a bleeding wound, **characterized by** the steps of:
providing a swab (10, 110, 210), a carrier material, and a molecular sieve material;
applying said carrier material to a contact portion of said swab (10, 110, 210);
applying said molecular sieve material to said contact portion of said swab (10, 110, 210); and
inserting said swab (10, 110, 210) with said molecular sieve material into said bleeding wound to facilitate contact between said molecular sieve material and blood.
